# EUROPEAN PATENT SPECIFICATION

(11) **EP 4 288 409 B1**
(45) Date of publication and mention of the grant of the patent: **05.08.2026**
(21) Application number: 21702151.8
(22) Date of filing: 07.02.2021
(51) Int. Cl.: C07C 213/02, C07C 217/28

(54) **COMPOSITION CONTAINING PHENALKAMINE AND METHOD FOR PREPARING SAME**
ZUSAMMENSETZUNG MIT PHENALKAMIN UND VERFAHREN ZUR HERSTELLUNG DAVON
COMPOSITION CONTENANT DE LA PHÉNALKAMINE ET SON PROCÉDÉ DE PRÉPARATION

(43) Date of publication of application: 13.12.2023
(73) Proprietor: Evonik Operations GmbH, 45128 Essen (DE)
(72) Inventor: FAN, Zhaoxin, Shanghai 201599 (CN); MAO, Xiulong, Shanghai 200030 (CN); ZHOU, Limin, Shanghai 201112 (CN); PENG, Qiubai, Shanghai 201112 (CN)
(74) Representative: Evonik Patent Association
(86) International application number: PCT/CN2021/075773
(87) International publication number: WO 2022/165796

(56) References cited:
- CN-A- 104 262 589
- SU-A1- 1 608 195
- GUANGZHOU SUPE CHEMICAL CO LTD: "CN 104 262 589 A - Substance table", CN 104 262 589 A, 1 January 2015 (2015-01-01), pages 1 - 18, XP093178009, Retrieved from the Internet <URL:https://content2.cas.org/v1/AUTH_9a355bb5cefd4c378bde0d541c6a11ce/patentpak-cdr-pdfplus-27/patent/73623918_1676858400.pdf?temp_url_sig=1b5960164f6f151db7aa67207eea9ec6c7bfdbbf&temp_url_expires=1719218483&inline>
- GAO NAN ;: "Non-ionic self-emulsifying water-borne epoxy curing agent having Gemini surfactant structure", CN104262589 A, 1 January 2015 (2015-01-01), pages 1 - 3, XP055823566

## Description

### Field

The present disclosure relates to a composition comprising a phenalkamine, and a method for preparing the composition.

### Background

Mannich base reactions are well-known. Mannich base compounds are products based on reaction of an aldehyde, generally formaldehyde, a phenolic compound, and an organic amine. Various forms of phenolic compounds, aldehydes, and amines have been proposed. Mannich base products are known to be used for curing epoxy resins.

Phenalkamines are a class of Mannich bases obtained by reacting phenolic compounds, aldehydes, and polyamines. Recently, phenalkamines based on cardanol have been synthesized and manufactured industrially. These chemicals are synthesized from a cardanol-containing extract derived from cashew nutshell liquid (sometimes shortened as "CNSL"), an aldehyde compound, such as formaldehyde, and a polyamine. As source of cardanol and cardol, cashew nut shell liquid is an abundant, sustainable, low cost product obtained as a byproduct of the cashew processing industry.

Traditionally, synthesis of phenalkamines uses ethylenediamine (EDA) and diethylenetriamine (DETA) as the polyamine. Phenalkamines are good epoxy resin curing agents for room temperature or low temperature curing applications.

US 6,262,148 B1 teaches curing agents based on Mannich base reaction products obtained by reacting cardanol with aromatic or alicyclic polyamine and aldehyde compounds.

CN 104262589 A discloses epoxy curing agents with a Gemini surfactant structure, prepared via a Mannich-type reaction of alkylphenols, polyamines, and formaldehyde, wherein the phenolic hydroxy functionality is positioned *para* to alkyl substituents containing at least one saturated carbon-carbon bond.

SU 1608195 A1 is directed to epoxy compositions comprising a block oligomer of epoxy resin, oligodiethylene glycol sebacate and an arylaliphatic amine curing agent.

WO 2019207079 A1 discloses a substituted cardanol with its benzene ring having at least two different groups. Each group has at least one hydrogen atom linked to an amine group. One group further has ether moieties. When used as curing agent, the substituted cardanol could allow to produce a bright cured composition that can be easily colored with dyes. The substituted cardanol has an inbuilt accelerating property and can reduce or avoid addition of accelerators.

Still, the market expects curing agents that could achieve fast curing rates and result in flexible and ductile epoxy products.

### Summary

One objective of the present disclosure is to provide a composition, which, when used as component of a curing agent and combined with epoxy resin, can realize fast curing and result in an epoxy resin with high flexibility and high elongation.

This objective of the present disclosure is achieved by providing a composition comprising a phenalkamine, which is represented by formula (I): 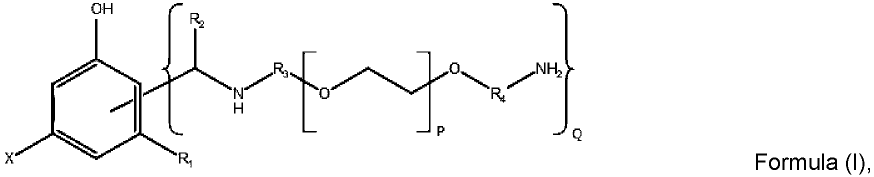 wherein, X is H or OH; R₁ is an aliphatic chain with more than 8 carbon atoms with at least one unsaturated bond; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P and Q are, independent of each other, an integer.

Preferably, Q is 1, 2, or 3.

Preferably, P is an integer not less than 1 and not larger than 50.

Preferably, R₁ is C₁₅H₃₁₋ₘ or C₁₇H₃₅₋ₘ, wherein m is 0, 2, 4, or 6.

Preferably, R₂ is H or CH₃.

Preferably, the composition further comprises a first polymer, which is represented by formula (II): 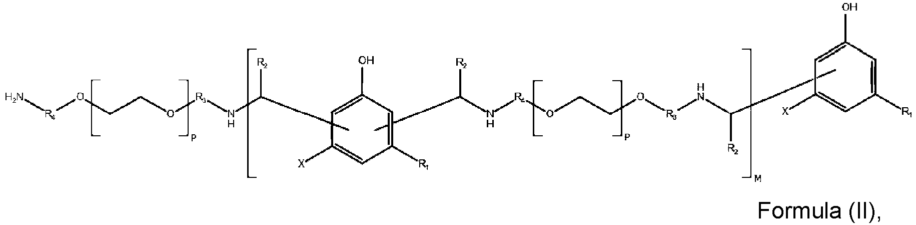 wherein, X is H or OH; R₁ is an aliphatic chain with more than 8 carbon atoms; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P is an integer; M is an integer not less than 1.

Preferably, the composition further comprises a second polymer, which is represented by formula (III): 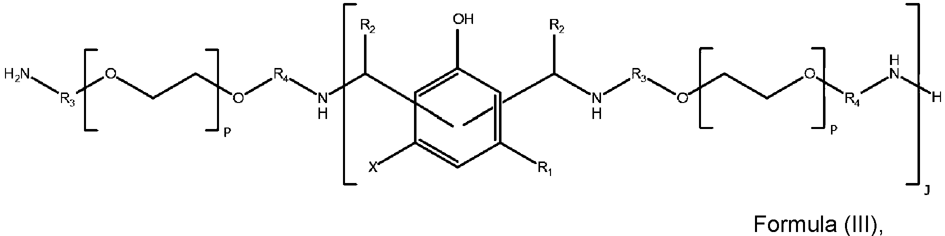 wherein, X is H or OH; R₁ is an aliphatic chain with more than 8 carbon atoms; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P is an integer; J is an integer not less than 2.

Preferably, the composition further comprises one or more additives selected from fillers, reinforcing agents, coupling agents, toughening agents, defoamers, dispersants, lubricants, colorants, marking materials, dyes, pigments, IR absorbers, antistats, anti-blocking agents, nucleating agents, crystallization accelerators, crystallization delayers, conductivity additives, carbon black, graphite, carbon nanotubes, graphene, desiccants, de-molding agents, levelling auxiliaries, flame retardants, separating agents, optical lighteners, rheology additives, photochromic additives, softeners, adhesion promoters, anti-dripping agents, metallic pigments, stabilizers, metal glitters, metal coated particles, porosity inducers, glass fibers, nanoparticles, flow assistants, or combinations thereof.

Another objective of the present disclosure is to provide a method for preparing the composition comprising a phenalkamine represented by formula (I): 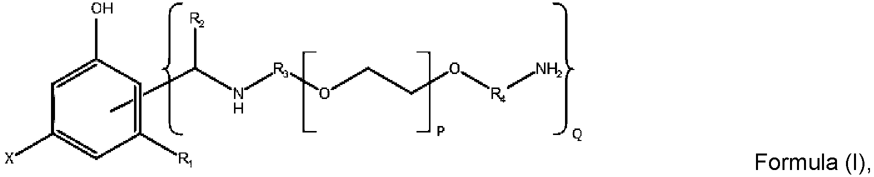 wherein, X is H or OH; R₁ is an aliphatic chain with more than 8 carbon atoms with at least one unsaturated bond; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; and P and Q are, independent of each other, an integer, the method comprising:
heating, to a temperature of 0 °C to 150 °C, a polyamine having at least two primary amino groups and at least one oxyethylene moiety, an alkyl phenol, and at least one aldehyde.

Preferably, Q is 1, 2, or 3.

Preferably, P is an integer not less than 1 and not larger than 50.

Preferably, the temperature is within 0 °C to 150 °C, more preferably 60 °C to 150 °C, still more preferably 70 °C to 130 °C.

Preferably, the polyamine is represented by formula (IV) as NH₂R₃(OCH₂CH₂)_{P}OR₄NH₂, wherein R₃ and R₄ are independently CH₂CH₂, CH₂CH₂CH₂, or CH₂CH₂CH₂CH₂; and P is an integer not less than 1 and not larger than 50.

Preferably, the aldehyde is formaldehyde or acetaldehyde.

Preferably, a molar ratio of the polyamine to the alkyl phenol is within a range of 0.1:1 to 10:1, preferably 0.2:1 to 5:1, more preferably 0.7:1 to 2:1.

Preferably, a molar ratio of the aldehyde to the alkyl phenol is within a range of 0.1:1 to 10:1, preferably 0.2:1 to 5:1, more preferably 0.7:1 to 2:1.

Compositions in the present disclosure provide two-component epoxy systems with excellent properties of fast cure, high flexibility, and high elongation, which are suitable for many applications such as road overlay, modified asphalt pavement, flexible adhesives, coatings, mortars, composites, etc.

### Detailed description

The following description is used merely for illustration but is not to restrict the scope of the present disclosure. The invention is defined by the claims.

The composition provided in the present disclosure comprises a phenalkamine, which is represented by formula (I): 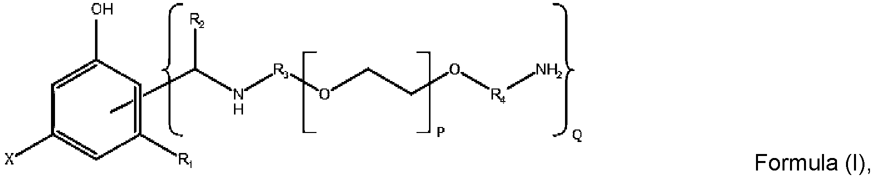 wherein, X is H or OH; R₁ is an aliphatic chain with more than 8 carbon atoms with at least one unsaturated bond; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P and Q are, independent of each other, an integer.

Preferably, Q is 1, 2, or 3.

Preferably, P is an integer not less than 1 and not larger than 50. More preferably, P is 1, 2, or 3.

Preferably, R₁ is C₁₅H₃₁₋ₘ or C₁₇H₃₅₋ₘ, wherein m is 0, 2, 4, or 6.

Preferably, R₂ is H or CH₃.

Preferably, the composition further comprises a first polymer, which is represented by formula (II): 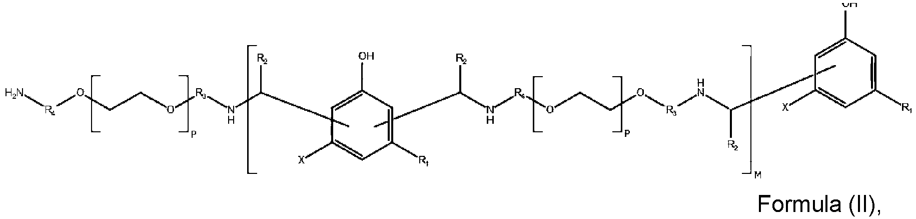 wherein, X is H or OH; R₁ is an aliphatic chain with more than 8 carbon atoms; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P is an integer; M is an integer not less than 1.

The first polymer has one amino group in one end and one partially reacted benzene ring in the other end. Preferably, the number M may be not larger than 20. Understandably, M depends on the conditions of the condensation, including the molar ratio of the reactants, the temperature under which the condensation is carried out, the concentrations of the reactants, and so on.

The first polymer is produced during condensation of alkyl phenol, aldehyde, and polyamine. The polyamine might bridge two or more aromatic rings through the CHR₂ moieties. Existence of the first polymer can be suggested by peaks in gel permeation chromatography analysis.

Preferably, the composition further comprises a second polymer, which is represented by formula (III): 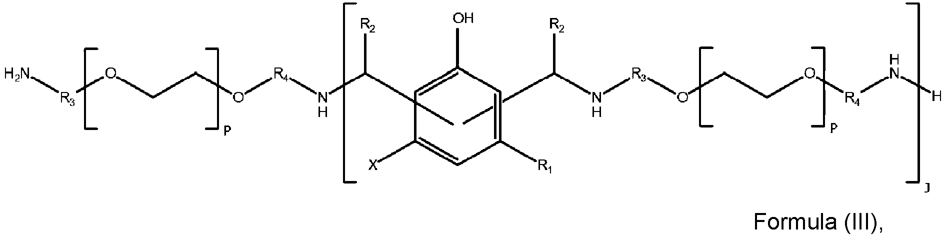 wherein, X is H or OH; R₁ is an aliphatic chain with more than 8 carbon atoms; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P is an integer; J is an integer not less than 2.

The second polymer has amino groups in both ends. Preferably, the number J may be not larger than 20. Understandably, J depends on the conditions of the condensation, including the molar ratio of the reactants, the temperature under which the condensation is carried out, the concentrations of the reactants, and so on.

The second polymer is produced during condensation of alkyl phenol, aldehyde, and polyamine. The polyamine might bridge two or more aromatic rings through the CHR₂ moieties. Existence of the second polymer can be suggested by peaks in gel permeation chromatography analysis.

The composition further comprises one or more additives selected from fillers, reinforcing agents, coupling agents, toughening agents, defoamers, dispersants, lubricants, colorants, marking materials, dyes, pigments, IR absorbers, antistats, anti-blocking agents, nucleating agents, crystallization accelerators, crystallization delayers, conductivity additives, carbon black, graphite, carbon nanotubes, graphene, desiccants, de-molding agents, levelling auxiliaries, flame retardants, separating agents, optical lighteners, rheology additives, photochromic additives, softeners, adhesion promoters, anti-dripping agents, metallic pigments, stabilizers, metal glitters, metal coated particles, porosity inducers, glass fibers, nanoparticles, flow assistants, or combinations thereof.

The composition can be prepared as a reaction product of a polyamine, an alkyl phenol, and an aldehyde.

### Polyamine

According to the present disclosure, the polyamine has at least two primary amino groups and at least one oxyethylene moiety. The oxyethylene moieties are connected to the primary amino groups through bivalent alkyl groups. Preferably, the polyamine is represented by formula (IV) as H₂NR₃(OCH₂CH₂)_{P}OR₄NH₂, wherein P is an integer not less than 1 and R₃ and R₄ are independently C₂H₄, C₃H₆, or C₄H₈. More preferably, the integer P is not larger than 50. Most preferably, P is 1, 2, or 3. More preferably, R₃ and R₄ are independently CH₂CH₂ or CH₂CH₂CH₂. Still more preferably, the polyamine includes H₂NCH₂CH₂CH₂O(CH₂CH₂O)₂CH₂CH₂CH₂NH₂, commercially available as Ancamine^{®} 1922A from Evonik Specialty Chemicals (Shanghai) Co., Ltd.

### Alkyl phenol

Alkyl phenol used in the present disclosure refers to a phenol with one or more alkyl groups on the benzene ring. The alkyl phenol includes a phenol having an aliphatic chain with more than 8 carbon atoms. Preferably, the alkyl phenol includes a phenol having an aliphatic chain with more than 12 carbon atoms.

The alkyl phenolincludes a phenol having an aliphatic chain with at least one unsaturated bond.

With limitation, alkyl phenol according to the present disclosure includes a nonylphenol, a cardanol, a cardol, or any mixture thereof.

Cardanol herein refers to a mixture of phenols which contain one hydroxyl group and differ in the number of unsaturated bonds in the aliphatic side chain in the meta-position. The structure of cardanol is shown as follows: 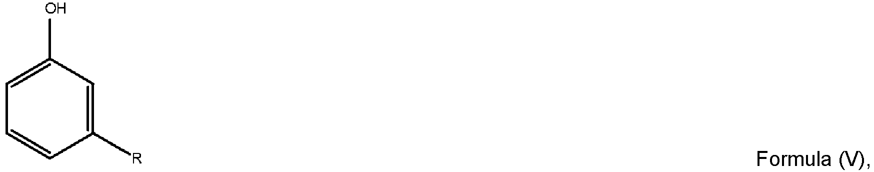 wherein R is a linear alkyl with 15 carbons containing 0 to 3 unsaturated bond(s) selected from the group consisting of -C₁₅H₃₁, -C₁₅H₂₉, -C₁₅H₂₇, and -C₁₅H₂₅; or a linear alkyl with 17 carbons containing 1 to 3 unsaturated bond(s) selected from the group consisting of -C₁₇H₃₃, -C₁₇H₃₁, and -C₁₇H₂₉.

Cardol has the following structure: 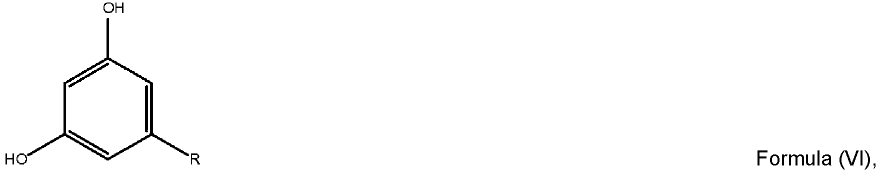 wherein R is a linear alkyl with 15 carbons containing 0 to 3 unsaturated bond(s) selected from the group consisting of -C₁₅H₃₁, -C₁₅H₂₉, -C₁₅H₂₇, and -C₁₅H₂₅; or a linear alkyl with 17 carbons containing 1 to 3 unsaturated bond(s) selected from the group consisting of -C₁₇H₃₃, -C₁₇H₃₁, and -C₁₇H₂₉.

Cardanol or cardol could be obtained with varying purities and chemical compositions from cashew nut shell liquid as a naturally occurring substance. They are commercially available from various manufacturers.

### Aldehyde

Aldehydes used to prepare the phenalkamine-containing composition provided in the present invention can be formaldehyde (in aqueous solution or as paraformaldehyde), acetaldehyde, propionaldehyde, butyraldehyde, heptaldehyde, hexaldehyde, 2-ethylhexanal, benzaldehyde, salicylaldehyde, any other aldehyde, or mixtures thereof. In a preferred embodiment, the aldehyde used in the present invention can be formaldehyde. These compounds are known in the art and are readily available from commercial sources or are easily made using known methods.

The composition of the present disclosure can be prepared according to the Mannich reaction conditions known in the art. The composition may be prepared by providing the aldehyde, the polyamine, and alkyl phenol described above, and reacting them via the Mannich reaction. Solvents such as benzene, toluene, or xylene can be used for removal of water produced during this reaction at an azeotropic distillation point. Nitrogen is also recommended for easing the water removal. The reaction may be conducted at a temperature from 0 to 150 °C, preferably 70 to 150 °C, or more preferably from 70 to 130 °C. In some embodiments, alkyl phenol and the polyamine are firstly mixed, and then the aldehyde is added into the resulting mixture. Time duration for adding the aldehyde can vary in the range of from 0.1 to 24 hours, preferably from 0.5 to 12 hours, or more preferably from 0.6 to 4 hours.

The molar ratio between the polyamine and the alkyl phenol is preferably 0.1:1 to 10:1, preferably, 0.2:1 to 5:1, more preferably, 0.7:1 to 2:1. The molar ratio between the aldehyde and the alkyl phenol is preferably 0.1:1 to 10:1, preferably 0.2:1 to 5:1, more preferably 0.7:1 to 2:1. The molar ratios between the polyamine and the alkyl phenol and between the aldehyde and the alkyl phenol has an influence of distribution of phenalkamines, the first polymer, and the second polymer in the condensates of the Mannich reaction.

### Curing agent

The composition as provided in the present disclosure could be used as a curing agent for epoxy resins. Besides the phenalkamine and polymer(s), the curing agent could include one or more components. Details regarding the components are described hereinafter.

The curing agent can further comprise at least one multifunctional amine. Multifunctional amine, as used herein, describes compounds with amine functionality and which contain two (2) or more amine hydrogen atoms.

Non-limiting examples of multifunctional amines that are within the scope of the present disclosure include, but are not limited to, an aliphatic amine, a cycloaliphatic amine, an aromatic amine; a Mannich base derivative of an aliphatic amine, a cycloaliphatic amine, or an aromatic amine; a polyamide derivative of an aliphatic amine, a cycloaliphatic amine, or an aromatic amine; an amidoamine derivative of an aliphatic amine, a cycloaliphatic amine, or an aromatic amine; an amine adduct derivative of an aliphatic amine, a cycloaliphatic amine, or an aromatic amine, and the like, or any combination thereof.

Preferably, more than one multifunctional amine is used in the compositions of the present disclosure. For example, the at least one multifunctional amine comprises an aliphatic amine and a Mannich base derivative of a cycloaliphatic amine. Also, the at least one multifunctional amine comprises one aliphatic amine and one different aliphatic amine.

Exemplary aliphatic amines include polyethyleneamines (ethylene diamine or EDA, diethylene triamine or DETA, triethylenetetraamine or TETA, tetraethylenepentamine or TEPA, pentaethylenehexamine or PEHA, and the like), polypropyleneamines, aminopropylated ethylenediamines, aminopropylated propylenediamines, 1,6-hexanediamine, 3,3,5-trimethyl-1,6-hexanediamine, 3,5,5-trimethyl-1,6-hexanediamine, 2-methyl-1,5-pentanediamine (commercially available as Dytek-A), and the like, or combinations thereof. Additionally, the poly(alkylene oxide) diamines and poly(alkylene oxide) triamines commercially available under the Jeffamine name from Huntsman Corporation, are useful in the present disclosure. Illustrative examples include, but are not limited to, Jeffamine^{®} D-230, Jeffamine^{®} D-400, Jeffamine^{®} D-2000, Jeffamine^{®} D-4000, Jeffamine^{®} T-403, Jeffamine^{®} EDR-148, Jeffamine^{®} EDR-192, Jeffamine^{®} C-346, Jeffamine^{®} ED-600, Jeffamine^{®} ED-900, Jeffamine^{®} ED-2001, and the like, or combinations thereof.

Cycloaliphatic and aromatic amines include, but are not limited to, 1,2-diaminocyclohexane, 1,3-diaminocyclohexane, 1,4-diaminocyclohexane, hydrogenated ortho-toluenediamine, hydrogenated meta-toluenediamine, metaxylylene diamine, hydrogenated metaxylylene diamine (referred to commercially as 1,3-BAC), isophorone diamine (IPDA), various isomers or norbornane diamine, 3,3'-dimethyl-4,4'-diaminodicyclohexyl methane, 4,4'-diaminodicyclohexyl methane, 2,4'-diaminodicyclohexyl methane, benzylated ethylene diamine, a mixture of methylene bridged poly(cyclohexyl-aromatic)amines, and the like, or combinations thereof. The mixture of methylene bridged poly(cyclohexyl-aromatic)amines is abbreviated as either MBPCAA or MPCA, and is described in U.S. Patent Number 5,280,091.

According to some embodiments of the present disclosure, the at least one multifunctional amine is an epoxidized 1,3-benzenedimethanamine (commercially available from Mitsubishi Gas Chemical Company as Gaskamine 328).

Mannich base derivatives can be made by the reaction of the above described aliphatic amines, cycloaliphatic amines, or aromatic amines with phenol or a substituted phenol and formaldehyde. An exemplary substituted phenol used to make Mannich bases with utility in the present disclosure is cardanol, which is obtained from cashew nut shell liquid. Alternatively, Mannich bases can be prepared by an exchange reaction of a multifunctional amine with a tertiary amine containing a Mannich base, such as tris-dimethylaminomethylphenol (commercially available as Ancamine^{®} K54 from Evonik Operations GmbH) or bis-dimethylaminomethylphenol.

Polyamide derivatives can be prepared by the reaction of an aliphatic amine, cycloaliphatic amine, or aromatic amine with a dimer fatty acid, or mixtures of a dimer fatty acid and a fatty acid. Amidoamine derivatives can be prepared by the reaction of an aliphatic amine, cycloaliphatic amine, or aromatic amine with fatty acids.

Amine adducts can be prepared by the reaction of an aliphatic amine, cycloaliphatic amine, or aromatic amine with an epoxy resin, for example, with the diglycidyl ether of bisphenol-A, the diglycidyl ether of bisphenol-F, or epoxy novolac resins. The aliphatic, cycloaliphatic, and aromatic amines also can be adducted with monofunctional epoxy resins, such as phenyl glycidyl ether, cresyl glycidyl ether, butyl glycidyl ether, other alkyl glycidyl ethers, and the like.

In another aspect of the present disclosure, the curing agent includes a co-curing agent. The co-curing agent may be an amidoamine curing agent, an aliphatic curing agent, a polyamide curing agent, a cycloaliphatic curing agent, or a Mannich base curing agent.

In some aspects of the present disclosure, a plasticizer is added to the curing agent.

To bring in more functionality or features to satisfy industrial requirements, the curing agent composition preferably includes additives. Additives are understood to mean substances which are added to alter the properties of the curing agent composition in the desired direction, for example to match viscosity, wetting characteristics, stability, reaction rate, blister formation, storability, or adhesion, and use properties, to the end application. Several additives are described, for example, in WO 99/55772, pp. 15-25.

Preferred additives are selected from the group consisting of fillers, reinforcing agents, coupling agents, toughening agents, defoamers, dispersants, lubricants, colorants, marking materials, dyes, pigments, IR absorbers, antistats, anti-blocking agents, nucleating agents, crystallization accelerators, crystallization delayers, conductivity additives, carbon black, graphite, carbon nanotubes, graphene, desiccants, de-molding agents, levelling auxiliaries, flame retardants, separating agents, optical lighteners, rheology additives, photochromic additives, softeners, adhesion promoters, anti-dripping agents, metallic pigments, stabilizers, metal glitters, metal coated particles, porosity inducers, glass fibers, nanoparticles, flow assistants, or combinations thereof.

The additive preferably constitutes a proportion of not greater than 90 wt. %, preferably not greater than 70 wt. %, more preferably not greater than 50 wt. %, still more preferably not greater than 30 wt. %, with respect to the total weight of curing agent.

For example, it is advantageous to add light stabilizers, for example sterically hindered amines, or other auxiliaries as described, for example, in a total amount of 0.05 % to 5 % by weight.

To produce the curing agent compositions of the present disclosure, it is additionally possible to add additives such as levelling agents, for example polysilicones, or adhesion promoters, for example those based on acrylate. In addition, still further components may optionally be present. Auxiliaries and additives used in addition may be chain transfer agents, plasticizers, stabilizers and/or inhibitors.

In some cases, the curing agent composition preferably includes an antioxidant additive. The antioxidant might include one or more of the structural units selected from sterically hindered phenols, sulfides, or benzoates. Here, in sterically hindered phenols, the two ortho-hydrogen atoms are substituted by compounds which are not hydrogen and preferably carry at least 1 to 20, particularly preferably 3 to 15, carbon atoms and are preferably branched. Benzoates also carry, preferably in the ortho position relative to the OH group, substituents which are not hydrogen and carry particularly preferably 1 to 20, more preferably, 3 to 15, carbon atoms, which are preferably branched.

In still another embodiment, if needed, one or more catalysts are preferably introduced to the curing agent composition, preferably as a part of the curing agent composition, to promote the reaction of the epoxide groups of epoxy resins and amine groups of the curing agent composition. Useful catalysts that may be introduced to the curing agent composition include Ancamide^{®} products available from Evonik Operations GmbH and products marketed as "Accelerators" available from Huntsman Corporation. One exemplary catalyst is piperazine-base Accelerator 399 available from Huntsman Corporation. When utilized, such catalysts preferably comprise between 0 and about 10 percent by weight of the total adhesive composition.

Preferably, a curing accelerator could be added into the curing agent composition for speeding up the curing process when the curing agent is mixed with epoxy resin. The curing accelerator includes one or more selected from tris-(dimethylaminomethyl) phenol, benzyl dimethylamine, various isomers of nonyl phenol, triethanolamine, or N-(3-aminopropyl) iminodiethanol.

Other additives or ingredients could be present in the system depending on the end application or environment to which the system is used.

Preferably, the curing agent composition according to the present disclosure comprises the above specified components.

### Epoxy resin in two-component epoxy system

The phenalkamine-containing composition of the present disclosure could be used with epoxy compounds already known in the art, to form a two-component epoxy composition.

Useful epoxy compounds are a multitude of those known for this purpose that contain more than one epoxide group, preferably two epoxide groups, per molecule. These epoxide compounds are preferably either saturated or unsaturated. They are preferably aliphatic, cycloaliphatic, aromatic, or heterocyclic, and have hydroxyl groups. They preferably contain such substituents that do not cause any side reactions under the mixing or reaction conditions, for example alkyl or aryl substituents, ether moieties and the like. They are preferably glycidyl ethers which derive from polyhydric phenols, especially bisphenols and novolac, and which have molar masses based on the number of epoxide groups ME ("epoxide equivalent weights", "EV value") between 100 and 1500 g/eq, but especially between 150 and 250 g/eq.

Examples of polyhydric phenols include: resorcinol, hydroquinone, 2,2-bis(4-hydroxyphenyl)propane (bisphenol A), bis(4-glycidyloxyphenyl)methane (bisphenol E), isomer mixtures of dihydroxydiphenylmethane (bisphenol F), 4,4'-dihydroxydiphenylcyclohexane, 4,4'-dihydroxy-3,3'-dimethyldiphenylpropane, 4,4'-dihydroxydiphenyl, 4,4'-dihydroxybenzophenone, bis(4-hydroxyphenyl)-1,1-ethane, bis(4-hydroxyphenyl)-1,1-isobutane, 2,2-bis(4-hydroxy-tert-butylphenyl)propane, bis(2-hydroxynaphthyl)methane, 1,5-dihydroxynaphthalene, tris(4-hydroxyphenyl)methane, bis(4-hydroxyphenyl) ether, bis(4-hydroxyphenyl) sulphone inter alia, and the chlorination and bromination products of the aforementioned compounds, for example tetrabromobisphenol A. Very particular preference is given to using liquid diglycidyl ethers based on bisphenol A and bisphenol F having an epoxide equivalent weight of 150 to 200 g/eq. It is also possible to use polyglycidyl ethers of polyols, for example ethane-1,2-diol diglycidyl ether, propane-1,2-diol diglycidyl ether, propane-1,3-diol diglycidyl ether, butanediol diglycidyl ether, pentanediol diglycidyl ether (including neopentyl glycol diglycidyl ether), hexanediol diglycidyl ether, diethylene glycol diglycidyl ether, dipropylene glycol diglycidyl ether, higher polyoxyalkylene glycol diglycidyl ethers, for example higher polyoxyethylene glycol diglycidyl ethers and polyoxypropylene glycol diglycidyl ethers, co-polyoxyethylene-propylene glycol diglycidyl ethers, polyoxytetramethylene glycol diglycidyl ethers, polyglycidyl ethers of glycerol, of hexane-1,2,6-triol, of trimethylolpropane, of trimethylolethane, of pentaerythritol or of sorbitol, polyglycidyl ethers of oxyalkylated polyols (for example of glycerol, trimethylolpropane, pentaerythritol, inter alia), diglycidyl ethers of cyclohexanedimethanol, of bis(4-hydroxycyclohexyl)methane and of 2,2-bis(4-hydroxycyclohexyl)propane, polyglycidyl ethers of castor oil, triglycidyl tris(2-hydroxyethyl)isocyanurate.

Further useful components A) include: poly(N-glycidyl) compounds obtainable by dehydrohalogenation of the reaction products of epichlorohydrin and amines such as aniline, n-butylamine, bis(4-aminophenyl)methane, m-xylylenediamine or bis(4-methylaminophenyl)methane. The poly(N-glycidyl) compounds also include triglycidyl isocyanurate, triglycidylurazole and oligomers thereof, N,N'-diglycidyl derivatives of cycloalkyleneureas and diglycidyl derivatives of hydantoins inter alia.

In addition, it is also possible to use polyglycidyl esters of polycarboxylic acids which are obtained by the reaction of epichlorohydrin or similar epoxide compounds with an aliphatic, cycloaliphatic or aromatic polycarboxylic acid such as oxalic acid, succinic acid, adipic acid, glutaric acid, phthalic acid, terephthalic acid, tetrahydrophthalic acid, hexahydrophthalic acid, naphthalene-2,6-dicarboxylic acid and higher diglycidyl dicarboxylates, for example dimerized or trimerized linolenic acid. Examples are diglycidyl adipate, diglycidyl phthalate and diglycidyl hexahydrophthalate.

Mention should additionally be made of glycidyl esters of unsaturated carboxylic acids and epoxidized esters of unsaturated alcohols or unsaturated carboxylic acids. In addition to the polyglycidyl ethers, it is possible to use small quantities of monoepoxides, for example methyl glycidyl ether, butyl glycidyl ether, allyl glycidyl ether, ethylhexyl glycidyl ether, long-chain aliphatic glycidyl ethers, for example cetyl glycidyl ether and stearyl glycidyl ether, monoglycidyl ethers of a higher isomeric alcohol mixture, glycidyl ethers of a mixture of C12 to C13 alcohols, phenyl glycidyl ether, cresyl glycidyl ether, p-tert-butylphenyl glycidyl ether, p-octylphenyl glycidyl ether, p-phenylphenyl glycidyl ether, glycidyl ethers of an alkoxylated lauryl alcohol, and also monoepoxides such as epoxidized monounsaturated hydrocarbons (butylene oxide, cyclohexene oxide, styrene oxide), in proportions by mass of up to 30 % by weight, preferably 10 % to 20 % by weight, based on the mass of the polyglycidyl ethers.

Useful epoxide compounds preferably include glycidyl ethers and glycidyl esters, aliphatic epoxides, diglycidyl ethers based on bisphenol A, bisphenol E and/or bisphenol **F,** and glycidyl methacrylates. Other examples of such epoxides are triglycidyl isocyanurate, mixtures of diglycidyl terephthalate and triglycidyl trimellitate (trade name: ARALDIT PT 910 and 912, Huntsman), glycidyl esters of versatic acid, 3,4-epoxycyclohexylmethyl 3,4'-epoxycyclohexanecarboxylate (ECC), ethylhexyl glycidyl ether, butyl glycidyl ether, pentaerythrityl tetraglycidyl ether, and other Polypox products having free epoxide groups. It is also possible to use mixtures of the epoxide compounds mentioned.

Particularly preferred epoxide components are polyepoxides based on bisphenol A diglycidyl ether, bisphenol E diglycidyl ether, bisphenol F diglycidyl ether, 4,4'-methylenebis[N,N-bis(2,3-epoxypropyl)aniline], hexanediol diglycidyl ether, butanediol diglycidyl ether, trimethylolpropane triglycidyl ether, propane-1,2,3-triol triglycidyl ether, pentaerythritol tetraglycidyl ether and diglycidyl hexahydrophthalate.

According to the present disclosure, it is also possible with preference to use mixtures of these epoxide compounds in the epoxy resin.

The epoxy resin may be in various forms, such as, a crystalline form, a powdered form, a semi-solid form, a liquid form, etc. For the liquid form, the epoxy resin may be dissolved in a solvent, for example, water. Preferably, the epoxy resin is in a liquid form, to facilitate the mixing process.

The phenalkamine-containing composition of the present disclosure may be used in various applications, for example, as water-proofing material for architecture, as coatings such as anticorrosion coating, and in road paving and maintenance applications. In particular, the composition is suitable for use in road paving and maintenance applications such as tack coats, fog seals, slurry seals and micro-surfacing. The phenalkamine-containing composition can be supplied with conventional equipment commonly used for a two-component epoxy system. During onsite application, one part (asphalt composition containing phenalkamines) and the other part (epoxy resin) can be stored in two different tanks, mixed, and optionally mixed with other optional components in a curable asphalt composition such as aggregates, then applied to a substrate such as road pavement. Phenalkamine-containing composition provided in the present disclosure can shorten the curing process, bring good compatibility with asphalt, and enhance flexibility and mechanical strengths in road pavement application.

The present disclosure is illustrated by way of synthesis example and comparative examples hereinbelow.

### Examples

In the following examples, the materials or definitions used are listed as below.

Ancamine^{®} 1922 was 3,3'-(oxybis(2,1-ethane-diyloxy)) bis-1-propanamine, a diamine with oxyethylene moieties from Evonik (Shanghai) Specialty Chemicals Co., Ltd.

Jeffamine^{®} D230 and Jeffamine^{®} D400 from Huntsman Corporation were diamines with oxypropylene moieties. Jeffamine^{®} T403 from Huntsman Corporation was a triamine with oxypropylene moieties.

Refined cashew nutshell liquid HD-F170 from Huada Saigao (Beijing) Technology Co., Ltd was a cardanol with 85 wt. % purity.

37 wt.% formaldehyde aqueous solution from Sinopharm Chemical Reagent Co., Ltd. was used as source of formaldehyde.

Molar ratio was calculated taking the molar number of cardanol as unit (1).

D.E.R.^{™} 331 was diglycidyl ether of bisphenol A, from Olin Corporation, a liquid reaction product of epichlorohydrin and bisphenol A. D.E.R.^{™} 331 has an EEW of 182-192 g/mol.

To compare phenalkamine-containing composition provided in the present disclosure with commercially available curing agents, three Ancamine^{®} curing agents were tested.

Ancamine^{®} 2758 is a phenalkamine from Evonik Specialty Chemicals (Shanghai) Co., Ltd. The curing agent use level was 35 phr for D.E.R. 331.

Ancamine^{®} 2770 is a phenalkamine from Evonik Specialty Chemicals (Shanghai) Co., Ltd. The curing agent use level was 65 phr for D.E.R. 331.

Ancamine^{®} 1637 is a Mannich base curing agent from Evonik Specialty Chemicals (Shanghai) Co., Ltd. The curing agent use level was 26 phr for D.E.R. 331.

Amine hydrogen equivalent weight in g/mol, or AHEW, is calculated as molecular weight of the amine divided by the number of amine hydrogen atoms per molecule.

Epoxide group content, indicated by epoxide equivalent weight or EEW, is the ratio between the molecular weight of the epoxide and the number of epoxide groups.

When describing chemical composition of two-component epoxy system, curing agent use level is often referred to. It is a dosage of curing agent per hundred resin (phr). In the examples it is calculated as amount of phenalkamine per epoxy resin (D.E.R. 331) having an EEW of 187 g/mol.

The following protocols to test physical performance or properties of sample were used:
Viscosity was measured according to ASTM D445-83 by a Brookfield DV-II+Pro Viscometer at 25 °C. Thin film set time (TFST) was measured using a Beck-Koller Drying Recorder, in accordance with ASTM D5895.

Amine value was measured on Mettler titrator according to ASTM D 2074 (perchloric Acid Titration).

To evaluate molecular weight distribution of the synthesized curing agent, gas permeation chromatography (GPC) analysis was conducted. PLgel MIXED GPC columns from Agilent Technologies, Inc. were used. Flow rate was set to be 0.3 mL/min. Concentration of the analyte in a tetrahydrofuran (THF) eluent was 5 mg/mL. The injection volume was 20 µL. Column temperature and pump temperature were 40 °C. Polystyrene was used as the calibration standard. A refractive index (RI) detector was used.

Gardner color was measured according to ASTM D 1544-80.

Gloss was measured by BYK gloss meter according to ASTM D 523-85.

Drying time was tested on BY drying recorder, according to ASTM D5895. Stage 1 is set-to-touch time. Stage 2 is tack-free time. Stage 3 is dry-hard time. Stage 4 is dry-through time. Hardness was tested with Shore D tester according to GB/T2411.

Water spot resistance is also known as carbamation resistance, blushing resistance. It was measured according to internal test method, which is described as follows. After curing for a certain period, for example 1 day or 2 days, a water saturated cotton ball was placed on the coating surface then covered by water glass. Next day, coating surface appearance after cotton removed is evaluated by ranking from 1 to 5.

| | |
|---|---|
| 1 - Very bad | White surface |
| 2 - Bad | Slight whitening |
| 3 - Moderate | Hazy surface |
| 4 - Good | Visible contours |
| 5 - Very good | Glossy surface |

Tensile properties including tensile strength, elongation at break, and tensile modulus were measured according to GB/T 2567-2008.

Flexibility was measured according to ASTM D522.

Impact resistance was measured according to ASTM D2794.

Cross-cut test for adhesion between a coating and a coated surface of substrate was measured according to DIN EN ISO 2409.

Glass transition temperature also known as Tg was the value at 2nd scan measured by DSC, 0-200 °C, heating rate of 10 °C/min according to ASTM D 3418-82.

Gel time was measured by a Techne^{®} Gelation Timer from Cole-Parmer Instrument Company, LLC using a 150-gram mixture.

Pot life was determined by measuring the time duration needed for value of the viscosity of mixture to be twice of its initial value.

Appearance after 24 hours at room temperature was evaluated by visual observation.

Compositions prepared by synthesis examples, compositions prepared by comparative examples, and commercial curing agents were cured with standard liquid epoxy resin of EEW 187 according to their respective stoichiometry. D.E.R.^{™} 331 was used as liquid epoxy resin. It is a diglycidyl ether of bisphenol A from Olin Corporation. Use levels in the synthesis examples and comparative examples were shown in Table 1. All samples were conditioned at room temperature 21-25 °C for 24h minimum before test. Phenalkamine-containing compositions were mixed with D.E.R.^{™} 331 using a speed mixer for 2 minutes at 1,500 rpm and then applied on the test substrates or poured into test molds. After cure at a specific temperature for a time period, the specimens were tested according to standard methods.

### Synthesis Procedure

Cardanol and amines were charged in a 1-L flask. Increased temperature to 70-80 °C while stirring. 37 wt. % formaldehyde aqueous solution was added into the flask dropwise while the cardanol-amine mixture was being stirred. After addition of formaldehyde solution was complete, the content inside the flask was heated to 100 °C. The temperature was kept for 100 minutes. Then it was elevated to 120 °C for 30-50 minutes. Water was removed under reduced pressure. The organic mixture was discharged and cooled down for testing.

In the synthesis examples SE 1 through SE 5, Ancamine^{®} 1922 from Evonik Specialty Chemicals (Shanghai) Co., Ltd. was used as the polyamine. In comparative examples CE 1 through CE 3, poly(propylene glycol)-based polyetheramines such as Jeffamine^{®} D230, Jeffamine^{®} D400, Jeffamine^{®} T403 from Huntsman Corporation were used as the polyamines. Details of synthesis examples and comparative examples were shown in Table 1.

From GPC analysis, the synthesized amines with different molar ratio of cardanol: polyamine: aldehyde contain various components mainly in the form of condensates with one, two, or more cardanol moieties, each directly connected with one or two -CH₂NH- linkage. Their existence and contents were suggested by the individual peaks. The average peak molecular weights (Mₚ) of synthesized amines with a molar ratio of 1:1:1 and 1:1:1.5 (cardanol/polyamine/aldehyde) were respectively 584 (repeating number of cardanol moieties being ) and 1234. These results indicated that excessive aldehyde could lead to high level of condensation and increase the quantity of higher condensates.

Test results were shown in Table 2 and Table 3. There was little test data for comparative sample CE 3 due to its incomplete cure with epoxy resins under some test conditions. At curing condition 2, tensile property data were available. The tensile strength was 47.4 MPa. The elongation was 8.7 %. The modulus was 1357 MPa. Commercial curing agents Ancamine^{®} 2758, Ancamine^{®} 2770, and Ancamine^{®} 1637 were used to further evaluate performances of phenalkamine-containing compositions provided in the present disclosure. In Table 3, they were shortened as A2758, A2770, and A1637, respectively.

As shown in Table 2 and Table 3, phenalkamine-containing compositions in synthesis example has fast curing properties than compositions prepared from poly(propylene glycol)-based diamine in comparative examples CE 1 and CE 2 in terms of gel time and pot life, much faster drying time. It also showed faster hardness development at low temperature.

Regarding mechanical performances, amines in synthesis example showed good flexibility expressed in high elongation, excellent impact resistance and bending flexibility at -20 °C compared with traditional ethylene diamine modified phenalkamines (as in Ancamine^{®} 2758, Ancamine^{®} 2770) and polyethylene polyamine modified Mannich base (as in Ancamine^{®} 1637). Compositions in synthesis example also showed excellent adhesion, excellent carbamation resistance and glossy finish at low temperature, which is very suitable for applications in protective coatings. Tensile modulus, tensile strength, and Shore hardness can be improved by varying portion of formaldehyde during synthesis without compromising fast cure or excellent flexibility.

The phenalkamine-containing compositions also show a good resistance to carbamation, which can lead to glossy surface and is highly desired in many applications such as road overlay, modified asphalt pavement, flexible adhesives, coatings, mortars, and composites.

Fast curing properties and excellent flexibility are desired in applications in coatings, road overlay and pavement, which allow fast open to traffic and improve cracking resistance significantly. The phenalkamine-containing compositions prepared by synthesis examples are suitable for road overlay applications, as normally elongation higher than 30% is required according to ASTM C881/C881M-14.

**Table 1**

| **Examples** | **SE 1** | **SE 2** | **SE 3** | **SE 4** | **SE 5** | **CE 1** | **CE 2** | **CE 3** |
|---|---|---|---|---|---|---|---|---|
| **Raw material (g)** | | | | | | | | |
| Cardanol | 500.8 | 481.3 | 470.1 | 441.4 | 562.3 | 491.7 | 385.7 | 366.9 |
| Ancamine^{®} 1922A | 364.8 | 350.7 | 342.4 | 321.6 | 286.7 | - | - | - |
| Jeffamine^{®} D230 | - | - | - | - | - | 376.3 | - | - |
| Jeffamine^{®} D400 | - | - | - | - | - | - | 510.8 | - |
| Jeffamine^{®} T403 | - | - | - | - | - | - | - | 534.6 |
| Formaldehyde | 134.4 | 168.0 | 187.5 | 237.0 | 151.0 | 132.0 | 103.6 | 98.5 |
| Total weight | 1,000.0 | 1,000.0 | 1,000.0 | 1,000.0 | 1,000.0 | 1,000.0 | 1,000.0 | 1,000.0 |

| **Properties** | | | | | | | | |
|---|---|---|---|---|---|---|---|---|
| Molar ratio | 1:1:1 | 1:1:1.2 | 1:1:1.5 | 1:1:2.0 | 1:0.7:1 | 1:1:1 | 1:1:1 | 1:1:1 |
| Viscosity (mPa·s) | 290 | 590 | 1,070 | 11,500 | 620 | 355 | 422 | 934 |
| Amine value (mg KOH/g) | 208 | 206 | 205 | 204 | 165 | 196 | 130 | 207 |
| Color grade | 14.0 | 12.6 | 13.1 | 13.0 | 14.6 | 17 | 15.8 | 13.1 |
| Water (%) | 0.20 | 0.21 | 0.36 | 0.30 | 0.20 | 0.21 | 0.12 | 0.10 |
| AHEW (g/eq) | 178.0 | 199.0 | 214.7 | 272.9 | 260.1 | 191.1 | 288.2 | 170.1 |
| Use level (phr) | 95.2 | 106.4 | 114.8 | 145.9 | 139.1 | 102.2 | 154.1 | 91.0 |

**Table 2**

| **Item** | | **1** | **2** | **3** | **4** | **5** |
|---|---|---|---|---|---|---|
| Curing agent | | SE 1 | SE 2 | SE 3 | SE 4 | SE 5 |
| Initial mixing viscosity at 25 °C (mPa·s) | | 1,005 | 1,440 | 2,166 | 26,694 | 1,353 |
| Gel time at 25 °C (min.) | | 66.3 | 109.6 | 144.6 | 91.2 | 81.6 |
| Pot life at 25 °C (min.) | | 43 | 54 | 61 | 71 | 48 |
| Dry time at 23 °C (hour) | Phase I | 5.5 | 7 | 6.5 | 3.5 | 6.3 |
| | Phase II | 7.6 | 8.2 | 7.5 | 5 | 8 |
| | Phase III | 9.5 | 9.5 | 9 | 10 | 9.5 |
| | Phase IV | 11.5 | 11 | 11.5 | 11 | 11.2 |
| Shore Hardness D | | 37.9 | 60.7 | 62.6 | 55.6 | 31.2 |
| Flexibility at -20 °C (mm) | | <1 | <1 | <1 | <1 | <1 |
| Cross cut adhesion (0-best) | | 0 | 0 | 0 | 0 | 0 |
| Impact resistance (kg·cm) | forward | >100 | >100 | >100 | >100 | >100 |
| | reverse | >100 | >100 | >100 | >100 | >100 |
| 1^{st} Tensile strength (MPa) [1] | | 5.21 | 8.27 | 11.81 | 5.27 | 1.76 |
| 1^{st} Tensile modulus (MPa) | | 16.8 | 126.5 | 186 | 14.6 | 4.2 |
| 1^{st} Elongation (%) | | 88.5 | 85.0 | 94.0 | 63.0 | 55.0 |
| Tg 2^{nd} scan (°C) | | 24.3 | 29.0 | 27.7 | 26.4 | 27.7 |
| 2^{nd} Tensile strength (MPa) [2] | | 8.95 | 9.52 | 7.489 | 3.88 | 2.04 |
| 2^{nd} Tensile modulus (MPa) | | 53.71 | 24.62 | 37.58 | 10.16 | 4.45 |
| 2^{nd} Elongation (%) | | 97.2 | 89.3 | 88.2 | 53.8 | 64.0 |

| | | | | | | |
|---|---|---|---|---|---|---|
| N.A.: Not tested [1] curing condition: 24 hours at room temperature and 2 hours at 60 °C [2] curing condition: 24 hours at room temperature and 4 hours at 60 °C and 2 hours at 150 °C | | | | | | |

**Table 3**

| **Item** | | **1** | **2** | **3** | **4** | **5** | **6** |
|---|---|---|---|---|---|---|---|
| Curing agent | | SE 1 | CE 1 | CE 2 | A2758 | A2770 | A1637 |
| Initial viscosity at 25 °C (mPa·s) | | 1,005 | 1,224 | 1,602 | 1,760 | 1,800 | 4,050 |
| Gel time at 25 °C (min.) | | 66.3 | 505.1 | 523.3 | 19 | 20 | 61 |
| Pot life at 25 °C (min.) | | 43 | 107 | 119 | 16 | 16 | 50 |
| Dry time at 23 °C (hour) | Phase I | 5.5 | 22.5 | 17 | 1.5 | 1 | 0.75 |
| | Phase II | 7.6 | >24 | 20 | 2.5 | 3 | 1.5 |
| | Phase III | 9.5 | >24 | >24 | 2.8 | 7 | 2 |
| | Phase IV | 11.5 | >24 | >24 | 3.7 | 9.5 | 3.5 |
| Appearance after 24 hours at room temperature | | Good | Tacky | Tacky | Hazy | Good | Good |
| Hardness Shore D | | 37.9 | 69.4 | 53.9 | 84.5 | 80.8 | 86.9 |
| Flexibility at -20 °C (mm) | | <1 | <1 | <1 | >15 | 4 | >15 |
| Cross cut adhesion (0-best) | | 0 | 0 | 0 | 2 | 0 | 0 |
| Impact resistance (kg·cm) | forward | >100 | >100 | >100 | 10 | 40 | 10 |
| | reverse | >100 | >100 | >100 | <5 | <5 | <5 |
| 1^{st} Tensile strength (MPa) [1] | | 5.21 | 11.08 | 3.72 | 65 | 44.6 | 66 |
| 1^{st} Tensile modulus (MPa) | | 16.8 | 702.5 | 6.8 | 3,603 | 1,528 | 3,400 |
| 1^{st} Elongation (%) | | 88.5 | 86.9 | 99.2 | 3.0 | 5.8 | 2.5 |
| Tg 2^{nd} scan (°C) | | 24.3 | 36.4 | 35.5 | 69.5 | 73.6 | 109.1 |
| 2^{nd} Tensile strength (MPa) [2] | | 8.95 | 21.2 | 1.12 | N.A. | N.A. | N.A. |
| 2^{nd} Tensile modulus (MPa) | | 53.71 | 1144.09 | 2.96 | N.A. | N.A. | N.A. |
| 2^{nd} Elongation (%) | | 97.2 | 53.5 | 48.8 | N.A. | N.A. | N.A. |
| Shore D hardness at 10 °C | 1d | 20.4 | Soft | Soft | 82.0 | 52.0 | 83.1 |
| | 2d | 39.6 | Soft | Soft | 83.0 | 76.0 | 85.2 |
| | 3d | 39.9 | 25.5 | 20.8 | 83.3 | 77.8 | 85.2 |
| | 7d | 39.7 | 67.2 | 52.5 | 83.8 | 78.6 | 86.7 |
| Carbamation resistance at 10 °C | 1d | 5 | Tacky | Tacky | 2 | 1 | 2 |
| | 2d | 5 | 5 | Tacky | 3 | 3 | 2 |

| | | | | | | | |
|---|---|---|---|---|---|---|---|
| N.A.: Not tested [1] curing condition: 24 hours at room temperature and 2 hours at 60 °C [2] curing condition: 24 hours at room temperature and 4 hours at 60 °C and 2 hours at 150 °C | | | | | | | |

## Claims

1. A composition comprising a phenalkamine represented by formula (I): 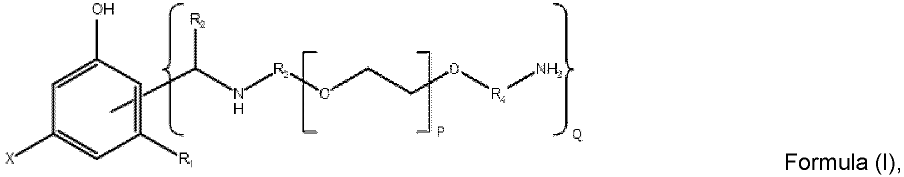 wherein, X is H or OH; R₁ is an aliphatic chain with more than 8 carbon atoms with at least one unsaturated bond; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; and P and Q are, independent of each other, an integer.

2. The composition of Claim 1, wherein Q is 1, 2 or 3.

3. The composition of Claim 1, wherein P is an integer not less than 1 and not larger than 50.

4. The composition of Claim 1, wherein R₁ is C₁₅H₃₁₋ₘ or C₁₇H₃₅₋ₘ, wherein m is 0, 2, 4, or 6.

5. The composition of Claim 1, wherein R₂ is H or CH₃.

6. The composition of Claim 1, further comprising a first polymer represented by formula (II): 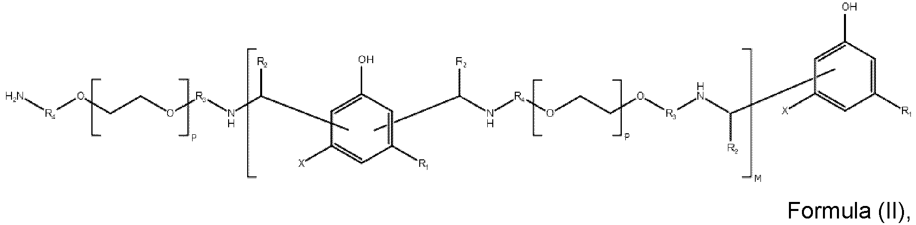 wherein, X is H or OH; R₁ is an aliphatic chain with more than 8 carbon atoms; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P is an integer; M is an integer not less than 1.

7. The composition of Claim 1, further comprising a second polymer represented by formula (III): 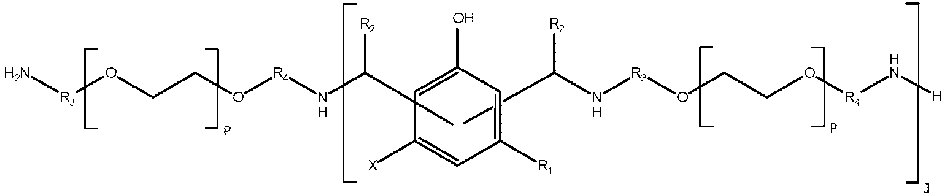 Formula (III), wherein, X is H or OH; R₁ is an aliphatic chain with more than 8 carbon atoms ; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; P is an integer; J is an integer not less than 2.

8. The composition of Claim 1, further comprising one or more additives selected from fillers, reinforcing agents, coupling agents, toughening agents, defoamers, dispersants, lubricants, colorants, marking materials, dyes, pigments, IR absorbers, antistats, anti-blocking agents, nucleating agents, crystallization accelerators, crystallization delayers, conductivity additives, carbon black, graphite, carbon nanotubes, graphene, desiccants, de-molding agents, levelling auxiliaries, flame retardants, separating agents, optical lighteners, rheology additives, photochromic additives, softeners, adhesion promoters, anti-dripping agents, metallic pigments, stabilizers, metal glitters, metal coated particles, porosity inducers, glass fibers, nanoparticles, flow assistants, or combinations thereof.

9. A method for preparing a composition comprising a phenalkamine represented by formula (I): 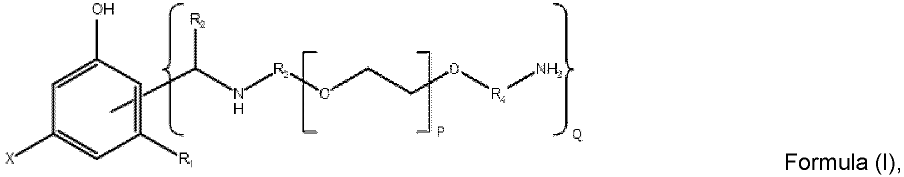 wherein, X is H or OH; R₁ is an aliphatic chain with more than 8 carbon atoms with at least one unsaturated bond; R₂ is H, a C1-C10 alkyl, phenyl, or a C5-C6 cycloaliphatic group; R₃ and R₄ are independently CH₂CH₂, CH₂CH₂CH₂, or CH₂CH₂CH₂CH₂; and P and Q are, independent of each other, an integer, the method comprising:
heating, to a temperature of 0 °C to 150 °C, a polyamine having at least two primary amino groups and at least one oxyethylene moiety, an alkyl phenol, and at least one aldehyde.

10. The method according to Claim 9, wherein the temperature is within 0 °C to 150 °C, preferably 60 °C to 150 °C, more preferably 70 °C to 130 °C.

11. The method according to Claim 9, wherein the polyamine is represented by formula (IV) as NH₂R₃(OCH₂CH₂)_{P}OR₄NH₂, wherein R₃ and R₄ are independently CH₂CH₂, CH₂CH₂CH₂, or CH₂CH₂CH₂CH₂; and P is an integer not less than 1 and not larger than 50.

12. The method according to Claim 9, wherein the aldehyde is formaldehyde or acetaldehyde.

13. The method according to Claim 9, wherein a molar ratio of the polyamine to the alkyl phenol is within a range of 0.1:1 to 10:1, preferably 0.2:1 to 5:1, more preferably 0.7:1 to 2:1.

14. The method according to Claim 9, wherein a molar ratio of the aldehyde to the alkyl phenol is within a range of 0.1:1 to 10:1, preferably 0.2:1 to 5:1, more preferably 0.7:1 to 2:1.

## Patentansprüche

1. Zusammensetzung umfassend ein durch Formel (I) dargestelltes Phenalkamin: 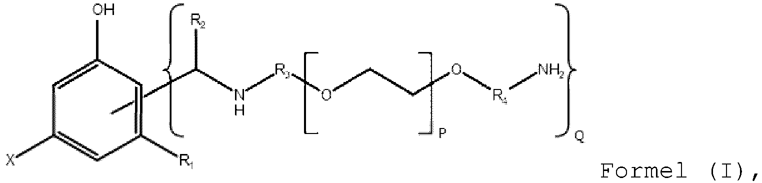 wobei X H oder OH ist; R₁ eine aliphatische Kette mit mehr als 8 Kohlenstoffatomen mit wenigstens einer ungesättigten Bindung ist; R₂ H, ein C1-C10-Alkyl, Phenyl oder eine C5-C6-cycloaliphatische Gruppe ist; R₃ und R₄ unabhängig CH₂CH₂, CH₂CH₂CH₂ oder CH₂CH₂CH₂CH₂ sind; und P und Q unabhängig voneinander eine ganze Zahl sind.

2. Zusammensetzung nach Anspruch 1, wobei Q 1, 2 oder 3 ist.

3. Zusammensetzung nach Anspruch 1, wobei P eine ganze Zahl von nicht kleiner als 1 und nicht größer als 50 ist.

4. Zusammensetzung nach Anspruch 1, wobei R₁ C₁₅H₃₁₋ₘ oder C₁₇H₃₅₋ₘ ist, wobei m 0, 2, 4 oder 6 ist.

5. Zusammensetzung nach Anspruch 1, wobei R₂ H oder CH₃ ist.

6. Zusammensetzung nach Anspruch 1, ferner umfassend ein durch Formel (II) dargestelltes erstes Polymer: 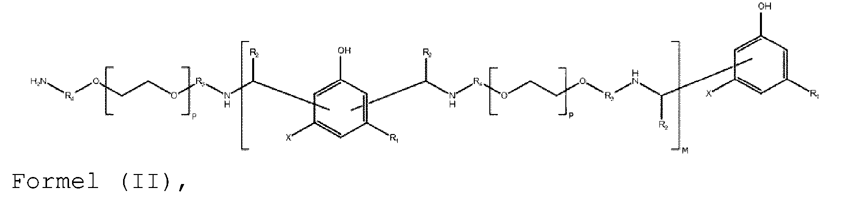 wobei X H oder OH ist; R₁ eine aliphatische Kette mit mehr als 8 Kohlenstoffatomen ist; R₂ H, ein C1-C10-Alkyl, Phenyl oder eine C5-C6-cycloaliphatische Gruppe ist; R₃ und R₄ unabhängig CH₂CH₂, CH₂CH₂CH₂ oder CH₂CH₂CH₂CH₂ sind; P eine ganze Zahl ist; M eine ganze Zahl von nicht kleiner als 1 ist.

7. Zusammensetzung nach Anspruch 1, ferner umfassend ein durch Formel (III) dargestelltes zweites Polymer: 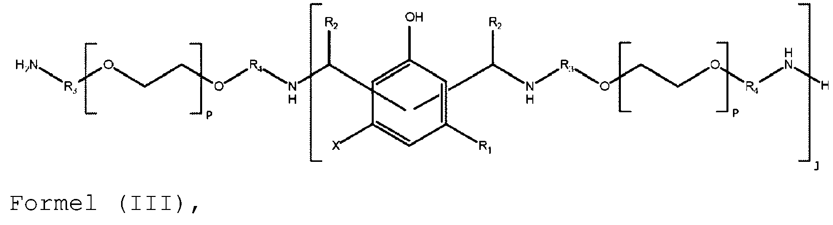 wobei X H oder OH ist; R₁ eine aliphatische Kette mit mehr als 8 Kohlenstoffatomen ist; R₂ H, ein C1-C10-Alkyl, Phenyl oder eine C5-C6-cycloaliphatische Gruppe ist; R₃ und R₄ unabhängig CH₂CH₂, CH₂CH₂CH₂ oder CH₂CH₂CH₂CH₂ sind; P eine ganze Zahl ist; J eine ganze Zahl von nicht kleiner als 2 ist.

8. Zusammensetzung nach Anspruch 1, ferner umfassend einen oder mehrere Zusatzstoffe ausgewählt aus Füllstoffen, Verstärkungsmitteln, Kopplungsmitteln, Schlagfestmachern, Entschäumern, Dispergiermitteln, Schmiermitteln, Farbmitteln, Markierungsmaterialien, Farbstoffen, Pigmenten, IR-Absorbern, Antistatika, Antiblockiermitteln, Keimbildnern, Kristallisationsbeschleunigern, Kristallisationsverzögerern, Leitfähigkeitszusatzstoffen, Ruß, Graphit, Kohlenstoffnanoröhrchen, Graphen, Trockenmitteln, Entformungsmitteln, Verlaufmitteln, Flammhemmern, Trennmitteln, optischen Aufhellern, Rheologiezusatzstoffen, photochromen Zusatzstoffen, Weichmachern, Haftförderern, Antitropfmitteln, Metallpigmenten, Stabilisatoren, Metall-Glittern, metallbeschichteten Partikeln, porositätsinduzierenden Mitteln, Glasfasern, Nanopartikeln und Fließhilfsmitteln und Kombinationen davon.

9. Verfahren zur Herstellung einer Zusammensetzung, die ein durch Formel (I) dargestelltes Phenalkamin umfasst: 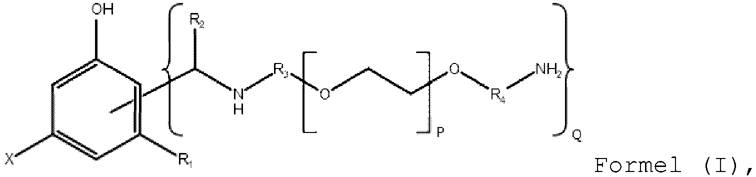 wobei X H oder OH ist; R₁ eine aliphatische Kette mit mehr als 8 Kohlenstoffatomen mit wenigstens einer ungesättigten Bindung ist; R₂ H, ein C1-C10-Alkyl, Phenyl oder eine C5-C6-cycloaliphatische Gruppe ist; R₃ und R₄ unabhängig CH₂CH₂, CH₂CH₂CH₂ oder CH₂CH₂CH₂CH₂ sind; und P und Q unabhängig voneinander eine ganze Zahl sind, wobei das Verfahren umfasst:
Erhitzen eines Polyamins mit wenigstens zwei primären Aminogruppen und wenigstens einem Oxyethylenrest, eines Alkylphenols und wenigstens eines Aldehyds auf eine Temperatur von 0 °C bis 150 °C.

10. Verfahren nach Anspruch 9, wobei die Temperatur innerhalb von 0 °C bis 150 °C, vorzugsweise 60 °C bis 150 °C, bevorzugter 70 °C bis 130 °C, liegt.

11. Verfahren nach Anspruch 9, wobei das Polyamin durch Formel (IV) als NH₂R₃(OCH₂CH₂)_{P}OR₄NH₂ dargestellt wird, wobei R₃ und R₄ unabhängig CH₂CH₂, CH₂CH₂CH₂ oder CH₂CH₂CH₂CH₂ sind und P eine ganze Zahl von nicht kleiner als 1 und nicht größer als 50 ist.

12. Verfahren nach Anspruch 9, wobei das Aldehyd Formaldehyd oder Acetaldehyd ist.

13. Verfahren nach Anspruch 9, wobei ein Molverhältnis des Polyamins zu dem Alkylphenol in einem Bereich von 0,1:1 bis 10:1, vorzugsweise 0,2:1 bis 5:1, bevorzugter 0,7:1 bis 2:1, liegt.

14. Verfahren nach Anspruch 9, wobei ein Molverhältnis des Aldehyds zu dem Alkylphenol in einem Bereich von 0,1:1 bis 10:1, vorzugsweise 0,2:1 bis 5:1, bevorzugter 0,7:1 bis 2:1, liegt.

## Revendications

1. Composition comprenant une phénalkamine représentée par la formule (I) : 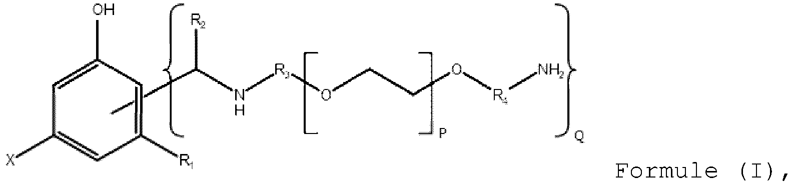 dans laquelle X est H ou OH ; R₁ est une chaîne aliphatique comportant plus de 8 atomes de carbone avec au moins une liaison insaturée ; R₂ est H, un alkyle en C1-C10, phényle, ou un groupe cycloaliphatique en C5-C6 ; R₃ et R₄ sont indépendamment CH₂CH₂, CH₂CH₂CH₂, ou CH₂CH₂CH₂CH₂ ; et P et Q sont, indépendamment l'un de l'autre, un entier.

2. Composition selon la revendication 1, dans laquelle Q est 1, 2 ou 3.

3. Composition selon la revendication 1, dans laquelle P est un entier non inférieur à 1 et non supérieur à 50.

4. Composition selon la revendication 1, dans laquelle R₁ est C₁₅H₃₁₋ₘ ou C₁₇H₃₅₋ₘ, où m est 0, 2, 4, ou 6.

5. Composition selon la revendication 1, dans laquelle R₂ est H ou CH₃.

6. Composition selon la revendication 1, comprenant en outre un premier polymère représenté par la formule (II) : 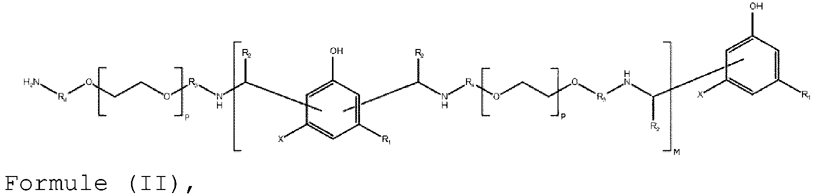 dans laquelle X est H ou OH ; R₁ est une chaîne aliphatique comportant plus de 8 atomes de carbone ; R₂ est H, un alkyle en C1-C10, phényle, ou un groupe cycloaliphatique en C5-C6 ; R₃ et R₄ sont indépendamment CH₂CH₂, CH₂CH₂CH₂, ou CH₂CH₂CH₂CH₂ ; P est un entier ; M est un entier non inférieur à 1.

7. Composition selon la revendication 1, comprenant en outre un deuxième polymère représenté par la formule (III) : 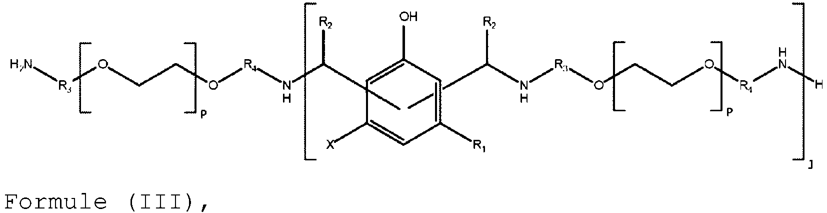 dans laquelle X est H ou OH ; R₁ est une chaîne aliphatique comportant plus de 8 atomes de carbone ; R₂ est H, un alkyle en C1-C10, phényle, ou un groupe cycloaliphatique en C5-C6 ; R₃ et R₄ sont indépendamment CH₂CH₂, CH₂CH₂CH₂, ou CH₂CH₂CH₂CH₂ ; P est un entier ; J est un entier non inférieur à 2.

8. Composition selon la revendication 1, comprenant en outre un ou plusieurs additifs sélectionnés parmi des charges, des agents de renforcement, des agents de couplage, des agents de ténacité, des antimousses, des dispersants, des lubrifiants, des matières colorantes, des matériaux de marquage, des colorants, des pigments, des absorbeurs d'IR, des antistatiques, des agents antiblocage , des agents de nucléation, des accélérateurs de cristallisation, des retardateurs de cristallisation, des additifs de conductivité, du noir de carbone, du graphite, des nanotubes de carbone, le graphène, des dessicatifs, des agents de démoulage, des auxiliaires de nivellement, des agents ignifugeants, des agents de séparation, des azurants optiques, des additifs de rhéologie, des additifs photochromiques, des assouplissants, des promoteurs d'adhérence, des agents anti-égouttement, des pigments métalliques, des stabilisants, des paillettes métalliques, des particules revêtues de métal, des agents d'induction de la porosité, des fibres de verre, des nanoparticules, des agents d'assistance à l'écoulement, ou des combinaisons correspondantes.

9. Procédé de préparation d'une composition comprenant une phénalkamine représentée par la formule (I) : 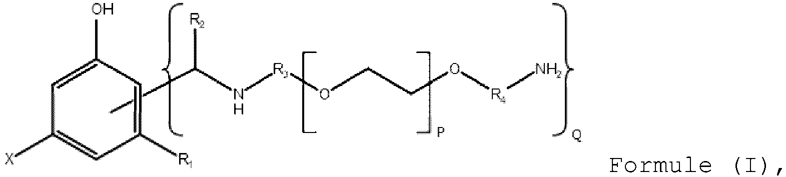 dans laquelle X est H ou OH ; R₁ est une chaîne aliphatique comportant plus de 8 atomes de carbone avec au moins une liaison insaturée ; R₂ est H, un alkyle en C1-C10, phényle, ou un groupe cycloaliphatique en C5-C6 ; R₃ et R₄ sont indépendamment CH₂CH₂, CH₂CH₂CH₂, ou CH₂CH₂CH₂CH₂ ; et P et Q sont, indépendamment l'un de l'autre, un entier, le procédé comprenant :
un chauffage, jusqu'à une température de 0 °C à 150 °C, d'une polyamine ayant au moins deux groupes amino primaires et au moins un groupement oxyéthylène, un alkylphénol, et au moins un aldéhyde.

10. Procédé selon la revendication 9, dans laquelle la température est dans la plage de 0 °C à 150 °C, préférablement 60 °C à 150 °C, plus préférablement 70 °C à 130 °C.

11. Procédé selon la revendication 9, dans lequel la polyamine est représentée par la formule (IV) en tant que NH₂R₃(OCH₂CH₂)_{P}OR₄NH₂, dans laquelle R₃ et R₄ sont indépendamment CH₂CH₂, CH₂CH₂CH₂, ou CH₂CH₂CH₂CH₂ ; et P est un entier non inférieur à 1 et non supérieur à 50.

12. Procédé selon la revendication 9, dans lequel l'aldéhyde est le formaldéhyde ou l'acétaldéhyde.

13. Procédé selon la revendication 9, dans lequel le rapport molaire de la polyamine sur l'alkylphénol est dans une plage de 0,1:1 à 10:1, de préférence 0,2:1 à 5:1, plus préférablement 0,7:1 à 2:1.

14. Procédé selon la revendication 9, dans lequel le rapport molaire de l'aldéhyde sur l'alkylphénol est dans une plage de 0,1:1 à 10:1, de préférence 0,2:1 à 5:1, plus préférablement 0,7:1 à 2:1.
